# EUROPÄISCHE PATENTSCHRIFT

(11) **EP 3 214 961 B1**
(45) Veröffentlichungstag und Bekanntmachung des Hinweises auf die Patenterteilung: **05.06.2019**
(21) Anmeldenummer: 15812941.1
(22) Anmeldetag: 01.11.2015
(51) Int. Cl.: A41D 19/00, A41D 19/015

(54) **HANDSCHUH**
GLOVE
GANT

(30) Priorität: 07.11.2014 DE 102014016525; 20.08.2015 DE 102015113861
(43) Veröffentlichungstag der Anmeldung: 13.09.2017
(73) Patentinhaber: IP Gloves GmbH, 52062 Aachen (DE)
(72) Erfinder: GLESER, Maxim, 30179 Hannover (DE); DIERS, Paul, 52062 Aachen (DE)
(74) Vertreter: Kudla, Christof
(86) Internationale Anmeldenummer: PCT/DE2015/100458
(87) Internationale Veröffentlichungsnummer: WO 2016/070868

(56) Entgegenhaltungen:
- EP-A2- 2 387 896
- WO-A1-00/19847
- WO-A1-2005/036997
- US-A- 5 566 394
- US-A1- 2011 030 121

## Beschreibung

Die Erfindung betrifft einen Handschuh, insbesondere einen Untersuchungs- oder Operationshandschuh oder einen Arbeitshandschuh, vorzugsweise aus einem flüssigkeitsundurchlässigen elastischem Material, der neben einem Handschuhkörper mit fünf Fingerräumen und einem Handflächenraum auch eine Stulpe aufweist, an deren Oberfläche genau eine Greifhilfe angeordnet ist. Weiterhin betrifft die Erfindung auch ein Verfahren zur Herstellung und die Verwendung des Handschuhs.

### Stand der Technik

Die WO 2005/036997 A1 beschreibt einen Handschuh mit an der Stulpe angeordneter Greifhilfe, wobei die Greifhilfe im den äußeren Knöchelbereich des Handgelenks bedeckenden Bereich angeordnet ist und/oder wobei die Greifhilfe sich über zumindest 180° des Umfangs der Stulpe erstreckt. Außerdem wird auch ein Handschuhpaar beschrieben, bei dem jeder Handschuh zumindest eine Greifhilfe aufweist und die Greifhilfen auf beiden Handschuhen auf aneinander angrenzenden Umfangsabschnitten der Stulpe vorgesehen sind und einen Winkelabstand von zumindest 30° aufweisen.

Die US 2011/0030121 A1 beschreibt medizinische Handschuhe mit Texturierungen an ausgewählten Stellen, insbesondere den Fingerspitzen, die dem Träger einen sicheren Halt bieten sollen. Darüber hinaus wird ein Handschuh offenbart, der vollständig mit Strukturierungen versehen ist und zudem eine Greifhilfe zum leichteren Ausziehen des Handschuhs aufweist, wobei die Greifhilfe im angezogenen Zustand des Handschuhs an der Innen- bzw. Außenseite des Handgelenks angeordnet ist.

Die US 5 566 394 A beschreibt Handschuhe mit einer knopfartigen Greifhilfe, die u.a. unterhalb des Daumens im Stulpenbereich angeordnet sein kann.

Die AT 505 712 A1 beschreibt drei verschiedene Ausführungsformen von Handschuhen. Die erste Ausführungsform betrifft Handschuhe mit an der Stulpe angeordneter Greifhilfe, wobei das Zugelastizitätsmodul des Greifhilfenmaterials größer als 10 MPa (bevorzugt größer als 100 MPa) oder doppelt so hoch (bevorzugt zehnmal so hoch) wie das es Stulpenmaterials ist. Das Material der Greifhilfe ist demnach unterschiedlich zu dem Material der Stulpe.
Die zweite Ausführungsform betrifft Handschuhe mit zumindest einer Greifhilfe im Stulpenbereich, wobei die Greifhilfe aus demselben Material ist wie der Handschuhkörper und mit diesem über eine kraftschlüssige Verbindung direkt im Bereich des Stulpenrandes oder an der Innenseite des Handschuhs verbunden ist. Eine weitere kraftschlüssige Verbindung befindet sich an der Handschuhaußenseite im oder oberhalb des Handgelenkbereichs.
Die dritte Ausführungsform betrifft Handschuhe mit einer Greifhilfe, die in einem Winkel von 30° bis 150° zum Stulpenabschluss angeordnet ist und durch mindestens einen und vorzugsweise zwei kraftschlüssige Fixierpunkte am Handschuh befestigt ist. Wie auch bei der ersten Ausführungsform ist das Zugelastizitätsmodul des Greifhilfenmaterials größer als 10 MPa (bevorzugt größer als 100 MPa) oder doppelt so hoch (bevorzugt zehnmal so hoch) wie das es Stulpenmaterials.

Die AT 413 190 B beschreibt einen Handschuh mit einer an der Stulpe angeordneten Greifhilfe. Die Greifhilfe ist dabei vom hinteren Rand der Stulpe beabstandet auf einem Umfangsteil der Stulpe vorgesehen, der sich auf der Handaußenseite dem Handrückenteil anschließt.

Die US 5 579 539 beschreibt Handschuhe mit einer im Stulpenbereich hervorstehenden Greifhilfe, die in einer bevorzugten Ausführungsform seitlich unterhalb des Daumens im Bereich des Musculus abductor pollicis longus angeordnet ist.

Die EP 1 675 486 B1 schützt ein Handschuhpaar sowie ein Verfahren zum Ausziehen eines Handschuhpaares, bei dem jeder Handschuh zumindest eine Greifhilfe aufweist, wobei die Greifhilfen auf beiden Handschuhen auf aneinander angrenzenden Umfangsabschnitten der Stulpe angeordnet sind und einen Winkelabstand von zumindest 135° aufweisen. Eine Greifhilfe eines Handschuhs ist dabei an der den äußeren Knöchelbereich des Handgelenks bedeckenden Außenseite der Stulpe angeordnet, die andere Greifhilfe ist aufgrund des vorgenannten Winkelabstands an anderer Stelle angeordnet.

Die DE 299 19 345 U1 beschreibt Schutzhandschuhe mit einem teilweisen Kunststoffüberzug, der zur Erhöhung der Abriebfestigkeit und der Griffigkeit Noppen aufweist. Die Handschuhe sind bevorzugt aus gewebtem oder gestricktem Material und weisen keine Greifhilfe auf.

### Aufgabe der Erfindung

Gegenüber dem bekannten Stand der Technik ist es die Aufgabe der vorliegenden Erfindung, einen alternativen Handschuh und ein Verfahren zu dessen Herstellung bereitzustellen, der nach dem Tragen auf einfache Weise auszuziehen ist, ohne dass der Träger während des Ausziehvorganges mit Verschmutzungen oder Kontaminationen auf der Außenseite des Handschuhs in Berührung kommt.

### Allgemeine Beschreibung der Erfindung

Die Erfindung löst die Aufgabe mit den Merkmalen der Ansprüche und insbesondere mit einem Handschuh, der genau eine auf dem Umfang der Stulpe angeordnete Greifhilfe aufweist, deren Oberfläche zumindest teilweise Strukturierungen, insbesondere Noppen und/oder Rillen, aufweist, wobei der Handschuh an zumindest zwei der insgesamt fünf Fingerräumen, die an die Finger einer Hand angepasst sind, jeweils im Wesentlichen an deren der Stulpe gegenüberliegenden Enden, die zur Aufnahme der Fingerspitzen und/oder Fingerkuppen vorgesehen sind, ebenfalls eine strukturierte Oberfläche bzw. Strukturierungen, insbesondere Noppen und/oder Rillen aufweist. Unter einer strukturierten Oberfläche wird für die Zwecke der Erfindung auch eine unregelmäßige Anordnung von Erhebungen bzw. Vertiefungen auf der betreffenden Stelle der Handschuhoberfläche verstanden. Erfindungsgemäße Handschuhe unterscheiden sich daher vor allem dadurch von aus dem Stand der Technik bekannten Handschuhen, dass sie im Stulpenbereich eine Greifhilfe mit zumindest anteilig strukturierter Oberfläche aufweisen und zusätzlich an zumindest zwei Fingerräumen jeweils an deren zur Aufnahme der Fingerspitzen eingerichteten Enden zumindest anteilig strukturierte Oberflächen aufweisen. Aufgrund dieser Gestaltung können erfindungsgemäße Handschuhe besonders gut und sicher von der Trägerhand entfernt werden, da die Greifhilfe mit ihrer zumindest anteilig strukturierten Oberfläche beim Ausziehvorgang von Fingerspitzen mit ebenfalls strukturierter Oberfläche ergriffen wird. Auf diese Weise erfolgt eine Interaktion der strukturierten Oberflächen im Fingerspitzenbereich mit der an der Oberfläche der Greifhilfe angeordneten strukturierten Oberfläche, z.B. ein "Ineinandergreifen" zweier genoppter Oberflächen, wodurch die Griffigkeit signifikant erhöht und das Risiko des Abrutschens von der Greifhilfe während des Ausziehens des Handschuhs signifikant reduziert wird.

Gemäß einer Ausführungsform kann ein erfindungsgemäßer Handschuh nur an der Greifhilfe und an zwei bis fünf Fingerräumen strukturierte Oberflächen aufweisen oder der erfindungsgemäße Handschuh kann gemäß einer alternativen Ausführungsform an seiner gesamten Oberfläche (womit für die Zwecke der Erfindung die Außenseite des Handschuhs gemeint ist, die im angezogenen Zustand keinen Kontakt zu der Haut des Trägers hat) eine strukturierte Oberfläche aufweisen.

Optional ist das Handschuhmaterial im Bereich der strukturierten Oberflächen dicker als an angrenzenden Bereichen ohne strukturierte Oberflächen mit gleicher Entfernung zum Rollrand. Der Vorteil einer erhöhten Materialdicke im Bereich der strukturierten Oberflächen liegt in der erhöhten Reißfestigkeit.

Im Einzelnen weist ein erfindungsgemäßer Handschuh die Merkmale des Anspruchs 1 auf.

Das Material, aus dem der Handschuh hergestellt ist, kann in Abhängigkeit von dem bezweckten Einsatzgebiet, d.h. der vom Handschuh zu erfüllenden Funktion, gewählt werden und kann insbesondere Acrylnitril, Nitril, Nitrilkautschuk, Polyethylen, Polyvinylchlorid, Vinyl, Polyvinylalkohol, Polyurethan, Gummi, Kautschuk, Naturkautschuk, Latex, Naturlatex, Neopren, Chloropren, Chloroprenkautschuk, Chloroprenlatex, Butylkautschuk, Fluorkautschuk oder Kombinationen dieser sein.

Die Stulpe des erfindungsgemäßen Handschuhs weist vorzugsweise an ihrem dem Handschuhkörper gegenüberliegenden Ende als Abschluss einen Rollrand auf. Der Rollrand kann eine beliebige Dicke aufweisen und besteht üblicherweise aus 5 bis 6 Lagen des aufgerollten Handschuhmaterials. Zwischen dem Rollrand und dem an den Handschuhkörper anschließenden Ende der Stulpe ist die genau eine Greifhilfe im angezogenen Zustand des Handschuhs seitlich des Handgelenks im Stulpenbereich angeordnet, wobei sie auch direkt an einem oder beiden Enden der Stulpe, d.h. direkt an oder in unmittelbarer Nähe zum Rollrand und/oder direkt an oder in unmittelbarer Nähe zu dem an den Handschuhkörper anschließenden Ende angeordnet sein kann. Bevorzugt ist eine Anordnung der genau einen Greifhilfe an oder in unmittelbarer Nähe zum Rollrand, da bei dieser Anordnung während des Ausziehvorgangs die Kraft beim Ziehen auf den Rollrand übertragen wird und die Handschuhe somit eine erhöhte Reißfestigkeit aufweisen. Ein weiterer Vorteil der Anordnung der Greifhilfe an oder in unmittelbarer Nähe zum Rollrand liegt darin begründet, dass ein Träger beim Beginn des Anziehvorgangs, d.h. beim Einführen einer Hand mit den Fingerspitzen voran in den Handschuh den Daumen naturgemäß eng an der Handinnenfläche anliegend hält, während er, sobald die Hand weiter in den Handschuh eingeführt ist, den Daumen seitlich neben die Handfläche bewegt. Bei einer an oder in unmittelbarer Nähe zum Rollrand angeordneten Greifhilfe besteht somit eine verringerte Gefahr, dass der Träger mit seinem Daumen in dem durch die Greifhilfe gebildeten Hohlraum "hängenbleibt" und der Handschuh dadurch beschädigt wird.

Optional weist die Greifhilfe des Handschuhs kleinere Dimensionen auf als der Fingerraum, der an einen Daumen angepasst ist, insbesondere einen (um beispielsweise 10-30 %) geringeren Umfang und/oder einen geringeren Durchmesser, beispielsweise einen um mindestens 20 % kleineren Durchmesser. Weiter optional ist die Länge der Greifhilfe senkrecht zu ihrem Umfang geringer als der durchschnittliche Abstand zwischen einer Daumenspitze und dem ersten Daumengelenk eines Erwachsenen. Die Länge der Greifhilfe (gemessen von ihrem Ansatz an der Stulpe bis zu ihrem gegenüberliegenden Ende) beträgt daher vorzugsweise weniger als 3 cm, insbesondere weniger als 2,5-2,7 cm. Durch die vorgenannten bevorzugten Dimensionen der Greifhilfe soll gewährleistet werden, dass der Daumen des Trägers beim Anziehen des Handschuhs nicht in die Greifhilfe gelangt, wodurch der Handschuh beschädigt werden könnte.

Erfindungsgemäß weist ein Handschuh genau eine Greifhilfe mit zumindest anteilig strukturierter Oberfläche auf. Diese ist im angezogenen Zustand des Handschuhs seitlich des Handgelenks im Stulpenbereich angeordnet. Eine mögliche Anordnung der Greifhilfe ist daher bei einer vertikalen Anordnung des Handschuhs mit nach oben weisenden Fingerräumen unterhalb des Fingerraums, der für die Aufnahme eines Daumens eingerichtet ist. Eine weniger bevorzugte alternative Anordnung der Greifhilfe ist bei einer vertikalen Anordnung des Handschuhs mit nach oben weisenden Fingerräumen unterhalb des Fingerraums, der für die Aufnahme eines kleinen Fingers eingerichtet ist. Erfindungsgemäß mit einer seitlich des Handgelenks angeordneten Greifhilfe eignet sich ein erfindungsgemäßer Handschuh insbesondere für eine beidseitige Tragbarkeit, d.h. der Handschuh kann sowohl an einer linken wie auch an einer rechten Hand getragen werden, wobei die Position der Greifhilfe dabei an jeder Hand gleich ist, nämlich entweder seitlich des Handgelenks unterhalb des Daumens oder seitlich des Handgelenks unterhalb des kleinen Fingers.

Für die Zwecke der Erfindung bedeutet die Formulierung "seitlich des Handgelenks" dabei im angezogenen Zustand eine Position an einer der beiden Schmalseiten des Handgelenks, wobei davon ausgegangen wird, dass jedes Handgelenk zwei Längsseiten und zwei Schmalseiten aufweist, wobei bei vertikaler Anordnung der Hand mit nach oben weisenden Fingerspitzen eine Längsseite unterhalb des Handrückens und die andere unterhalb der Handinnenfläche angeordnet ist und die Schmalseiten jeweils gegenüberliegend zwischen den beiden Längsseiten angeordnet sind.

Neben der beidseitigen Tragbarkeit erfindungsgemäßer Handschuhe mit einer seitlich des Handgelenks angeordneten Greifhilfe hat diese Anordnung darüber hinaus auch den Vorteil, dass sich die Handschuhe besser in größeren Stückzahlen, z.B. zu je 100 Stück, in Kartons verpacken lassen. Denn zum einen können Handschuhe mit entsprechend angeordneter Greifhilfe platzsparender verpackt werden, da durch die Greifhilfe keine zusätzliche Lage Handschuhmaterial entsteht. Zum anderen wird bei Handschuhen mit im angezogenen Zustand seitlich des Handgelenks angeordneter Greifhilfe die Greifhilfe bei einer Stapelung im Karton nicht plattgedrückt.

Als weiteren Vorteil einer im angezogenen Zustand des Handschuhs seitlich des Handgelenks im Stulpenbereich angeordneten Greifhilfe haben Messungen mit einem Kraftmesser ergeben, dass bei der vorgenannten Anordnung der Greifhilfe ein um 4 % verringerter Kraftaufwand des Handschuhträgers nötig ist, um die Handschuhe nach der Benutzung durch Ergreifen an der Greifhilfe von der Hand abzuziehen.

Gemäß einer Ausführungsform weisen erfindungsgemäße Handschuhe an der Greifhilfe, insbesondere an den Übergängen zwischen Greifhilfe und restlichem Handschuh, eine im Vergleich zum restlichen Handschuh erhöhte Wandstärke auf. Diese wird dadurch erzeugt, dass sich beim Eintauchen der Formkörper in ein Tauchbad eine oder mehrere Einkerbungen auf der Erhebung, durch welche die Greifhilfe erzeugt wird, befinden. In dieser zumindest einen Einkerbung sammelt sich vermehrt die in dem Tauchbad befindliche Flüssigkeit, sodass beim anschließenden Trocknen der Handschuhe an den Positionen der Einkerbungen Bereiche mit erhöhter Wandstärke erzeugt werden. Durch die an dieser Stelle erhöhte Materialdicke weisen die entsprechenden Bereiche auch eine im Vergleich zum restlichen Handschuh veränderte Optik auf, da sie in der Regel dunkler sind als der restliche Handschuh. Dies bietet den zusätzlichen Vorteil einer optischen Signalwirkung, die den Träger daran erinnern soll, die Handschuhe zum Entfernen von der Hand nur an der Greifhilfe zu fassen.

Die Form der zumindest einen Greifhilfe ist variabel, wobei die Greifhilfe insbesondere im Wesentlichen knaufförmig, lappenförmig, bandförmig, zungenförmig, pyramidenförmig, kegelförmig, kegelstumpfförmig oder winkelförmig ausgebildet sein kann. Die Greifhilfe ist eine Erhebung auf der Oberfläche der Stulpe, die im angezogenen Zustand des Handschuhs, d.h. wenn der Handschuh an der Hand getragen wird, anders als der Rest des Handschuhs nicht eng an der Hand anliegt, sondern von der Oberfläche des Handschuhs absteht, insbesondere in Form eines Lappens, wobei insbesondere zwischen der Innenseite des Lappens und der Hand des Trägers ein Hohlraum gebildet wird. Abgesehen von den Ausmaßen unterscheidet sich der vorgenannte durch die Greifhilfe gebildete Hohlraum vorzugsweise nicht wesentlich von den Hohlräumen, die durch die fünf Fingerräume gebildet werden. Insbesondere weist der Handschuh im angezogenen Zustand keine Luftblasen, Kanten oder sonstige Unregelmäßigkeiten im Bereich der Greifhilfe auf.

Gemäß einer Ausführungsform hat die genau eine Greifhilfe die Form eines winklig auf dem Stulpenumfang angeordneten Lappens, wobei der Winkel beispielsweise ein spitzer Winkel oder ein rechter Winkel und insbesondere ein stumpfer Winkel ist, dessen einer Schenkel etwa senkrecht zu dem den Abschluss der Stulpe bildenden Rollrand verläuft und insbesondere zu diesem beabstandet ist, wobei der andere Schenkel ausgehend vom Scheitelpunkt in Richtung des Fingerraums verläuft, der für die Aufnahme eines Daumens eingerichtet bzw. an einen Daumen angepasst ist. Diese winklige Anordnung der Greifhilfe auf dem Umfang der Stulpe ist besonders ergonomisch, da der Handschuh zum Ausziehen besonders gut etwa im Scheitelpunkt der Greifhilfe mit dem Daumen und zumindest einem weiteren Finger ergriffen und von der Trägerhand abgezogen werden kann. Ein weiterer Vorteil einer Greifhilfe als winklig auf dem Umfang der Stulpe angeordneter Lappen liegt darin, dass sich die Greifhilfe beim Anziehen nicht ausdehnt und dadurch gut sichtbar und greifbar ist.

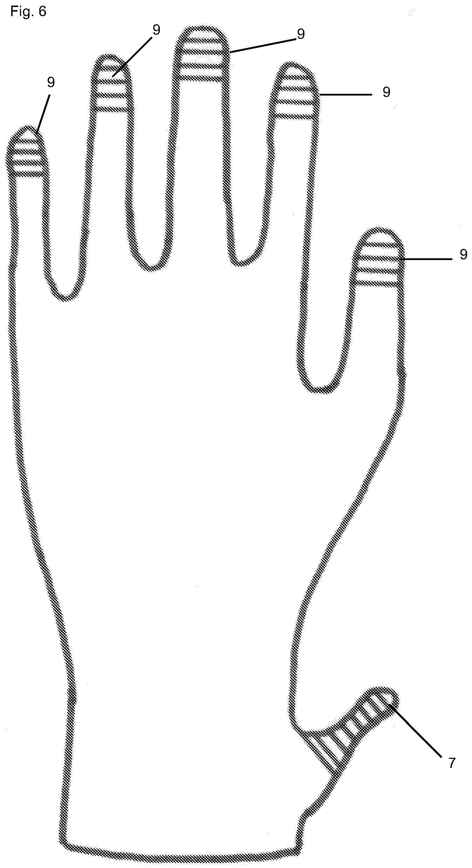

Gemäß einer bevorzugten Ausführungsform hat die Greifhilfe eine kegelstumpf-ähnliche Form und ist im angezogenen Zustand seitlich des Handgelenks, bevorzugt unterhalb des für die Aufnahme eines Daumens eingerichteten Fingerraums, angeordnet. Auf ihrer der Stulpe abgewandten Seite weist die Greifhilfe bevorzugt eine Mulde auf, wie diese z.B. in Fig. 6 gezeigt ist, in der die Materialdicke vorzugsweise erhöht ist.

Weiterhin betrifft die Erfindung auch ein Handschuhpaar, das aus zwei erfindungsgemäßen Handschuhen besteht. Diese zwei erfindungsgemäßen Handschuhe können identisch oder unterschiedlich sein.

Um sicherzustellen, dass der Träger beim Ausziehen des einen Handschuhs mit der den Handschuh ergreifenden anderen Hand nicht abrutscht, ist die Oberfläche der Greifhilfe eines erfindungsgemäßen Handschuhs zumindest teilweise strukturiert, wobei der erfindungsgemäße Handschuh vorzugsweise an der gesamten Greifhilfe eine strukturierte Oberfläche aufweist. Diese strukturierte Oberfläche weist bevorzugt Erhebungen und Vertiefungen auf, die insbesondere regelmäßig angeordnet sind, zum Beispiel insbesondere parallel verlaufende Rillen, Lamellen, Riffelungen oder regelmäßig beabstandete Rauten, Karos oder Noppen. Die vorgenannten Strukturierungen verleihen der Greifhilfe insbesondere nicht nur eine höhere Griffigkeit beim Anfassen, sondern auch eine erhöhte Standfestigkeit, welche das Ergreifen mit der jeweils anderen Hand erleichtert. Vorzugsweise beträgt die Fläche der strukturierten Oberfläche an der Greifhilfe mindestens 0,1 bis 0,5 cm², bevorzugter mindestens 1 cm² und besonders bevorzugt mindestens 2 cm².

In einer Ausführungsform weist die Greifhilfe zwei oder mehr Bereiche strukturierter Oberflächen auf, die durch Bereiche mit unstrukturierten Oberflächen voneinander getrennt sind. Dies bringt den Vorteil einer erhöhten Reißfestigkeit mit sich, da insbesondere bei Handschuhen, bei denen die Greifhilfe unmittelbar an oder in der Nähe des den Stulpenabschluss bildenden Rollrands angeordnet ist, durch die nicht strukturierten Bereiche eine bessere Kraftübertragung auf den Rollrand erfolgt. Eine erhöhte Reißfestigkeit im Vergleich zu herkömmlichen Handschuhen mit oder ohne Greifhilfe erlaubt bei dafür zugelassenen Handschuhen eine häufigere Wiederbenutzung.

Neben der strukturierten Oberfläche an der Greifhilfe weisen erfindungsgemäße Handschuhe auch an zumindest zwei Fingerräumen jeweils zumindest eine strukturierte Oberfläche auf, insbesondere an dem Fingerraum, der zur Aufnahme eines Daumens eingerichtet ist, sowie an zumindest einem weiteren Fingerraum. Die strukturierte Oberfläche an jedem dieser Fingerräume ist dabei insbesondere an den jeweiligen dem Handflächenraum gegenüberliegenden Enden der Fingerräume, die zur Aufnahme der jeweils äußeren Fingerglieder eingerichtet sind, angeordnet. Dabei sind die Strukturierungen an den Fingerräumen entweder vollumfänglich oder nur teilumfänglich an den jeweiligen Enden der Fingerräume angeordnet. Im Falle einer nur teilumfänglich an den Enden der Fingerräumen angeordneten strukturierten Oberfläche kann diese an jedem Fingerraum eine oder mehrere Flächen mit strukturierter Oberfläche bilden, wobei mehrere strukturierte Oberflächen durch Oberflächen ohne Strukturierungen miteinander verbunden sind. Es ist bevorzugt, dass die Fläche einer jeden strukturierten Oberfläche an einem Fingerraum mindestens 0,1 bis 0,5 cm², bevorzugter mindestens 1 cm² und besonders bevorzugt mindestens 2 cm² groß ist.

Wie die strukturierte Oberfläche der Greifhilfe weist auch die strukturierte Oberfläche an den Fingerräumen vorzugsweise Erhebungen und Vertiefungen auf, die insbesondere regelmäßig angeordnet sind, zum Beispiel insbesondere parallel verlaufende Rillen, Riffelungen, Lamellen oder regelmäßig beabstandete Rauten, Karos oder Noppen.

Die strukturierte Oberfläche der Greifhilfe weist identische oder unterschiedliche Strukturierungen auf wie die strukturierte Oberfläche an den zumindest zwei Fingerräumen.
Bei jeweils identischen Strukturierungen, z.B. Noppen, an Greifhilfe und Fingerräumen ergreift ein Handschuhträger beim Ausziehen mit den genoppten Oberflächen an zumindest zwei Fingerräumen des einen Handschuhs die ebenfalls genoppte Oberfläche der Greifhilfe am anderen Handschuh. Die Strukturierungen greifen daher ineinander und ermöglichen auf diese Weise einen besonders stabilen Halt beim Ausziehen. Auch unterschiedliche Strukturierungen an der Greifhilfe und an den Fingerräumen erlauben jedoch bei entsprechender Abstimmung der Strukturierungen aufeinander einen stabilen Halt und somit ein sicheres Ausziehen der Handschuhe. Beispielsweise könnte bei unterschiedlichen Strukturierungen die strukturierte Oberfläche an den Fingerräumen Noppen aufweisen, die in eine gitterartige Strukturierung an der strukturierten Oberfläche der Greifhilfe eingreifen.

In einer Ausführungsform hat die strukturierte Oberfläche an den Fingerräumen und/oder die strukturierte Oberfläche an der Greifhilfe eine von dem restlichen Handschuh abweichende Farbe, insbesondere eine Signalfarbe, z.B. rot. Auf diese Weise wird der Träger durch die visuelle Besonderheit beim Ausziehen daran erinnert, die strukturierte Oberfläche der Greifhilfe mit den strukturierten Oberflächen der Fingerräume zu ergreifen und durch den optimalen Halt des Handschuhs zwischen beiden strukturierten Oberflächen ein Abrutschen und damit die Gefahr von Kontaminationen insbesondere des Unterarms oder des Handgelenks zu vermeiden.

Für eine beidseitige Tragbarkeit weisen erfindungsgemäße Handschuhe vorzugsweise an den Enden von zumindest zwei Fingerräumen, insbesondere dem an einen Daumen angepassten Fingerraum sowie zumindest einem weiteren Fingerraum, vollumfänglich eine strukturierte Oberfläche auf. Insbesondere weisen erfindungsgemäße Handschuhe an dem für den Daumen vorgesehenen Fingerraum bzw. an dessen Ende sowie an den zwei zu diesem Fingerraum benachbarten Fingerräumen (zur Aufnahme von Zeige- und Mittelfinger) vollumfänglich strukturierte Oberflächen auf. Besonders bevorzugt sind an den Enden eines jeden der fünf Fingerräume vollumfänglich identisch strukturierte Oberflächen angeordnet.

Für die Zwecke der Erfindung wird unter einer vollumfänglichen Anordnung der strukturierten Oberflächen an den Enden der Fingerräume auch die Anordnung von zwei um etwa 180° auf dem Umfang der Fingerräume beabstandeten strukturierten Oberflächen an den Enden der Fingerräume verstanden, die durch eine unstrukturierte Oberfläche miteinander verbunden sind.

Generell erfüllen die erfindungsgemäßen Handschuhe die Standards und Anforderungen, die an Handschuhe auf ihrem jeweiligen Einsatzgebiet gestellt werden. Beispielsweise weisen medizinische Handschuhe, z.B. Untersuchungshandschuhe oder chirurgische Handschuhe, einen AQL-Wert von max. 2,5 und insbesondere von 1,5 bis 2 oder unterhalb von 1,5 auf.

Insbesondere in Abhängigkeit des beabsichtigten Einsatzgebietes können erfindungsgemäße Handschuhe auch Benutzungshinweise und/oder Identifizierungszeichen aufweisen, die an beliebiger Stelle angebracht sind. Beispielsweise können direkt auf der Greifhilfe oder in dem an diese angrenzenden Bereich Benutzungshinweise angebracht sein, die den Träger daran erinnern sollen, den Handschuh zum Entfernen an der Greifhilfe zu erfassen.

Erfindungsgemäße Handschuhe haben im Handflächenbereich vorzugsweise eine Wanddicke von ca. 0,03 bis 0,15 mm, während ihre Wanddicke im Bereich der Fingerräume vorzugsweise 0,07 bis 0,2 mm beträgt. Auch ultradünne erfindungsgemäße Handschuhe halten dabei einer Zugkraft von mehr als 6 Newton stand, insbesondere einer an der Greifhilfe ansetzenden Zugkraft.

Die Erfindung betrifft weiterhin auch ein Verfahren zur Herstellung von Handschuhen mit den Merkmalen des Anspruchs 1.

In dem erfindungsgemäßen Verfahren werden die Handschuhe wie auch herkömmliche Handschuhe mit Hilfe von Formkörpern hergestellt, die in ein Tauchbad, z.B. mit oder aus Acrylnitril getaucht werden und anschließend getrocknet, vulkanisiert und z.B. mittels Druckluft von den Formkörpern entfernt werden.

Nach dem Eintauchen der Formkörper in das Tauchbad, dem sogenannten "dipping", was üblicherweise mit den Fingerspitzen der Modellhand voran erfolgt, werden die Formkörper vorzugsweise in der gleichen Position, d.h. mit den Fingerspitzen nach unten, trocknen gelassen. Auf diese Weise werden Handschuhe erzeugt, die im Bereich der Fingerspitzen eine erhöhte Materialdicke aufweisen. Alternativ können die Formkörper nach dem Eintauchen in das Tauchbad auch um 90-180° gedreht werden, sodass sich die in dem Tauchbad befindliche Flüssigkeit nicht an den Fingerspitzen der Modellhand sammelt, sondern zumindest zum Teil in entgegengesetzte Richtung, d.h. in Richtung der Stulpe fließt und sich somit besser verteilt. Auf diese Weise werden Handschuhe mit gleichmäßigerer Materialdicke erzeugt.

Eine Drehung der Formkörper nach dem Eintauchen in das Tauchbad, insbesondere um 90°, ist auch aus dem Grund sinnvoll, dass die Handschuhe nach der Trocknung besser von den Formkörpern abgestreift werden können. Da eine solche Drehung der Formkörper nach dem Trocknen nicht ohne Weiteres in derselben Produktionsstraße möglich ist, in der die Formkörper nach dem Eintauchen in das Tauchbad ohne Drehung trocknen gelassen werden, können die Formkörper erfindungsgemäß mittels eines Zwischenstücks auf der Produktionsstraße angeordnet werden. In dieser Ausführungsform weist das erfindungsgemäße Verfahren daher vor dem Eintauchen der Formkörper in das Tauchbad einen Schritt der Anordnung der Formkörper auf Zwischenstücken, die jeweils an der Produktionsstraße befestigt sind, auf.

Bei aus dem Stand der Technik bekannten Handschuhen mit Greifhilfe zeigt sich oftmals das Problem, dass diese in dem Bereich zwischen den Fingerräumen, die an einen Daumen und an einen Zeigefinger angepasst sind sowie in dem Bereich zwischen der Greifhilfe und dem Fingerraum, der an einen Daumen angepasst ist (beide Bereiche werden als "crotch area" bezeichnet), eine zu geringe Wandstärke haben, da die in dem Tauchbad befindliche Flüssigkeit, die beim Eintauchen der Formkörper in das Tauchbad an diesen haften bleibt, zum Teil von der Greifhilfe abgefangen wird, sodass zu wenig Flüssigkeit in die beiden vorgenannten als "crotch area" bezeichneten Bereiche fließt.

Um dieses Problem bei erfindungsgemäßen Handschuhen zu vermeiden, weisen die in dem erfindungsgemäßen Verfahren eingesetzten Formkörper vorzugsweise ein Reservoir für Flüssigkeit aus dem Tauchbad auf, in dem sich beim Eintauchen der Formkörper Flüssigkeit ansammelt. Nach dem Herausnehmen der Formkörper aus dem Tauchbad fließt diese Flüssigkeit durch gezielte Dreh-/ und/oder Kippbewegungen der Formkörper in den Bereich zwischen den Fingerräumen, die an einen Daumen und an einen Zeigefinger angepasst sind, sowie in den Bereich zwischen der Greifhilfe und dem Fingerraum, der an einen Daumen angepasst ist.

Alternativ oder zusätzlich dazu weisen die in dem erfindungsgemäßen Verfahren eingesetzten Formkörper im Vergleich zu aus dem Stand der Technik bekannten Formkörpern Bereiche mit präferierter Fließrichtung auf. Dies können z.B. Bereiche mit Neigungen sein, durch welche vermehrt nach dem Herausnehmen der Formkörper aus dem Tauchbad an den Formkörpern anhaftende Flüssigkeit in Richtung des Bereichs der beiden vorgenannten "crotch areas" fließt.
Die vorgenannten Bereiche mit präferierter Fließrichtung können jedoch auch durch eine gezielte Anordnung der strukturierten Oberflächen auf der Greifhilfe gebildet werden. Nach dem Eintauchen der Formkörper in das Tauchbad erfolgt die Trocknung üblicherweise mit bei vertikaler Haltung nach unten weisenden Fingerspitzen bzw. Fingerräumen. Aufgrund der Schwerkraft fließt daher an den Formkörpern anhaftende Flüssigkeit ebenfalls nach unten in Richtung der Fingerspitzen bzw. Fingerräume. An der Greifhilfe haftet die Flüssigkeit aufgrund der Strukturierungen auf der Greifhilfe besser an diesen an und sammelt sich aufgrund eines Rückstaus herabfließender Flüssigkeit bevorzugt im Bereich des Übergangs zwischen den Strukturierungen und den in Richtung des Rollrands liegenden angrenzenden unstrukturierten Bereichen. Durch gezielte Anordnung von Noppen oder anderen strukturierten Oberflächen auf der Greifhilfe kann daher erreicht werden, dass das an den Formkörpern anhaftende Material aus dem Tauchbad während des Abtropf- bzw. Trocknungsschritts gezielt umverteilt wird, wodurch die Materialdicke erfindungsgemäßer Handschuhe im Bereich der "crotch areas" im Vergleich zu Handschuhen mit Greifhilfe ohne Strukturierungen erhöht wird. Das Risiko der Entstehung von Löchern im Bereich der "crotch areas" kann somit durch gezielte Anordnung strukturierter Oberflächen auf der Greifhilfe minimiert werden, beispielsweise um zumindest 3% oder sogar um ca. 3-10 %, jeweils im Vergleich zu Handschuhen mit unstrukturierter Greifhilfe.

Für die vorgenannte gezielte Anordnung von strukturierten Oberflächen auf der Greifhilfe ist es jedenfalls notwendig, dass die Strukturierungen bei vertikaler Haltung mit nach unten weisenden Fingerspitzen bzw. Fingerräumen zumindest zum Teil oberhalb der "crotch area" zwischen der Greifhilfe und dem Fingerraum, der an einen Daumen angepasst ist, angeordnet sind, da das Material sonst nicht durch die Schwerkraft in Richtung der "crotch area" fließen kann. Beispielsweise können die Strukturierungen im Wesentlichen parabelförmig auf der Greifhilfe angeordnet sein, wobei der Scheitelpunkt der Parabel bei vertikaler Haltung des Handschuhs mit nach unten weisenden Fingerspitzen bzw. Fingerräumen oberhalb der "crotch area" zwischen der Greifhilfe und dem Fingerraum, der an einen Daumen angepasst ist, angeordnet ist.

Optional weisen die Formkörper beim Eintauchen in das Tauchbad auch eine oder mehrere Einkerbungen auf der Erhebung, durch welche die Greifhilfe erzeugt wird, auf. In dieser zumindest einen Einkerbung sammelt sich vermehrt die in dem Tauchbad befindliche Flüssigkeit, sodass beim anschließenden Trocknen der Handschuhe an den Positionen der Einkerbungen Bereiche mit erhöhter Wandstärke erzeugt werden. Da sich beim "dipping" durch die zumindest eine Einkerbung genug Material aus dem Tauchbad im Handgelenkbereich des Handschuhs sammelt, kann gemäß einer vorteilhaften Ausführungsform weniger viskoses Material, z.B. weniger viskoses Latex, als Flüssigkeit für das Tauchbad verwendet werden. In dieser Ausführungsform kann auch die nachfolgende Vulkanisation bei niedrigeren Temperaturen erfolgen als die Vulkanisation von aus dem Stand der Technik bekannten Handschuhen mit einer Greifhilfe, die keine Einkerbungen aufweist, beispielsweise bei ca. 5-10 % niedrigeren Temperaturen. Diese erniedrigten Temperaturen bewirken eine Kostenersparnis im Vulkanisationsschritt.

Im Unterschied zu herkömmlichen Herstellungsverfahren für Handschuhe zeichnet sich das erfindungsgemäße Herstellungsverfahren jedenfalls durch die Bereitstellung von Formkörpern aus, die die Geometrie einer Hand mit einem daran anschließenden im Wesentlichen zylinderförmigen Abschnitt haben, auf dessen Umfang zumindest eine Erhebung angeordnet ist, wobei an der Oberfläche der Erhebung zumindest anteilig Strukturierungen, insbesondere gleichmäßig angeordnete Strukturierungen, angeordnet sind, und wobei an zumindest zwei Fingern der Modellhand jeweils an deren der Handfläche gegenüberliegenden Enden ebenfalls Strukturierungen angeordnet sind. Diese bereitgestellten Formkörper werden anstelle von herkömmlichen Formkörpern in Form einer Hand mit glatter und im Wesentlichen ebener Oberfläche in bekannter Weise in ein Bad mit oder aus chemischen und/oder natürlichen Inhaltsstoffen getaucht, anschließend getrocknet und vulkanisiert, sowie optional gewaschen und gereinigt. Das Verfahren zum Herstellen erfindungsgemäßer Handschuhe zeichnet sich daher dadurch aus, dass die genau eine Greifhilfe mit ihrer zumindest anteilig strukturierten Oberfläche sowie die strukturierten Oberflächen an den Fingerräumen des Handschuhkörpers im formgebenden Prozess des Handschuhs miterzeugt werden.

Optional werden erfindungsgemäße Handschuhe während oder nach dem Eintauchen in das Tauchbad chloriert, wodurch die Stretcheigenschaften der Handschuhe verändert werden können. Nicht erfindungsgemäße Handschuhe weisen für eine Elongation von 50-100 % einen Stretch-Modulus von 2,1 MPa (± 40 %) auf, während erfindungsgemäße Handschuhe für eine entsprechende Elongation einen Modulus von bis zu 3,4 MPa (± 40 %) aufweisen können.

Das erfindungsgemäße Verfahren weist weiterhin einen Schritt des Trennens der Handschuhe von den Formkörpern auf, das sogenannte "stripping". Dieser Schritt kann komplett manuell erfolgen ("manuelles stripping") oder er umfasst das Abstreifen der fertigen Handschuhe von den Formkörpern mit Hilfe von zumindest zwei Abstreifern ("maschinelles stripping"). Diese Abstreifer, beispielsweise Bürsten oder Rollen, fahren dabei den Formkörper von der Stulpe in Richtung der Fingerspitzen der Modellhand ab, wobei der Handschuh bis auf die jeweilige Höhe der Abstreifer von dem Formkörper abgezogen wird. Dabei wird ein weiterer Vorteil der im angezogenen Zustand seitlich des Handgelenks angeordneten Greifhilfe deutlich: bei Handschuhen mit nur einer seitlich angeordneten Greifhilfe besteht nicht die Gefahr, dass die Abstreifer an der Greifhilfe hängen bleiben, wodurch der Handschuh beschädigt werden könnte, wie dies beispielsweise bei zwei ober- und unterhalb des Handgelenks gegenüberliegend angeordneten Greifhilfen der Fall sein kann.

Bevorzugt umfasst der Schritt des Trennens der Handschuhe von den Formkörpern auch ein Herunterblasen der Handschuhe von den Formkörpern mittels Druckluft. Dabei ist es bevorzugt, dass die Druckluft mittels zweier oder mehrerer Kanäle auf die Stulpe und auf die Greifhilfe geblasen wird, sodass sich die Handschuhe mit der Greifhilfe besser von den Formkörpern lösen.

Optional weist das erfindungsgemäße Verfahren vor dem Schritt des Trennens der Handschuhe von den Formkörpern einen Schritt auf, der als "beading" bezeichnet wird. Bei dem erfindungsgemäßen "beading" wird im Gegensatz zu herkömmlichen Verfahren der Rollrand nicht mittels einer verhältnismäßig großen Bürste, sondern mittels zweier kleinerer Bürsten aufgerollt, die beispielsweise einen um 10-50 % geringeren Durchmesser haben als bei herkömmlichen Verfahren im "beading" eingesetzte Bürsten. Der Vorteil zweier Bürsten mit geringerem Durchmesser besteht darin, dass diese im Bereich der Greifhilfe nicht auf unvulkanisiertes Material, z.B. auf unvulkanisiertes Nitril, treffen, wodurch der Handschuh reißen könnte.

Alternativ zu dem vorbeschriebenen Verfahren zum Herstellen erfindungsgemäßer Handschuhe kann der in dem Verfahren bereitgestellte Formkörper mit der Geometrie einer Hand und einem daran anschließenden zylinderförmigen Abschnitt statt der zumindest einen Erhebung auf dem Umfang des zylinderförmigen Abschnitts auch zumindest eine Vertiefung auf dessen Umfang aufweisen, wobei auf der Oberfläche der Vertiefung zumindest anteilig Strukturierungen, insbesondere gleichmäßig angeordnete Strukturierungen, die insbesondere wiederum Vertiefungen sind, angeordnet sind, und wobei an zumindest zwei Fingern der Modellhand jeweils an deren der Handfläche gegenüberliegenden Enden ebenfalls Strukturierungen in Form von Vertiefungen angeordnet sind. Auf diese Weise können die Handschuhe in dem erfindungsgemäßen Herstellungsverfahren beim Abziehen von der Modellhand einmal umgekrempelt, d.h. "auf links gedreht" werden, wodurch die Greifhilfe mit ihrer strukturierten Oberfläche beispielsweise lappenförmig von der Oberfläche des Handschuhs absteht.

Die Erfindung betrifft weiterhin auch die Verwendung erfindungsgemäßer Handschuhe, als Untersuchungs-, Operations- oder Arbeitshandschuh. Da die erfindungsgemäßen Handschuhe insbesondere zur Sicherstellung eines kontaminationsfreien Arbeitens vorgesehen sind, ist es vor dem Hintergrund einer guten Handhygiene auch vorgesehen, nach dem Entfernen der Handschuhe ein Desinfektionsmittel für die Hände zu verwenden. Die Erfindung betrifft daher auch ein Kit-of-parts, umfassend ein Paar erfindungsgemäßer Handschuhe sowie ein Handdesinfektionsmittel.

### Genaue Beschreibung der Erfindung

Die Erfindung wird nun genauer anhand von Beispielen und mit Bezug auf die Figuren beschrieben, in denen schematisch
- Figur 1 eine Hand mit einem erfindungsgemäßen Handschuh,
- Figur 2 den Ausziehvorgang eines erfindungsgemäßen Handschuhs,
- Figur 3 eine Hand mit einem weiteren erfindungsgemäßen Handschuh,
- Figur 4 eine Hand mit einem weiteren erfindungsgemäßen Handschuh,
- Figur 5 einen in einem erfindungsgemäßen Verfahren einsetzbaren Formkörper, und
- Figur 6 eine bevorzugte Ausführungsform eines erfindungsgemäßen Handschuhs zeigt.

In den Figuren bezeichnen gleiche Bezugsziffern funktionsgleiche Teile.

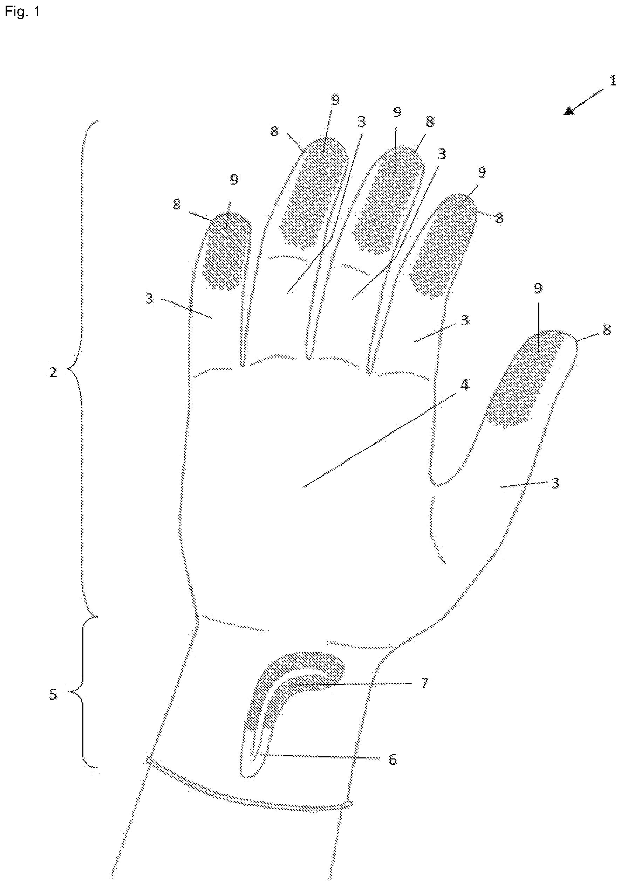

Figur 1 zeigt einen Handschuh 1 mit einem Handschuhkörper 2 und einer Stulpe 5. Der Handschuhkörper 2 weist fünf Fingerräume 3 auf, die jeweils mit dem Handflächenraum 4 verbunden sind. An ihren dem Handflächenraum 4 gegenüberliegenden Enden 8 weisen die Fingerräume 3 jeweils strukturierte Oberflächen 9 auf. Obwohl generell eine Anordnung der strukturierten Oberflächen 9 auf dem vollen Umfang der Fingerräume 3, zumindest im Bereich ihrer dem Handflächenraum 4 gegenüberliegenden Enden 8, bevorzugt ist, sind die strukturierten Oberflächen 9 in der gezeigten Ausführungsform nur teilumfänglich an den Enden 8 der Fingerräume 3 angeordnet. Die strukturierten Oberflächen 9 weisen jeweils regelmäßig angeordnete Noppen auf, die sich von den Enden 8 der Fingerräume 3 in Richtung des Handflächenraums 4 erstrecken, d.h. im angezogenen Zustand des Handschuhs an den Fingerkuppen der jeweils äußeren Fingerglieder bzw. auch der daran angrenzenden Fingerglieder zu liegen kommen.

An der Stulpe 5 ist eine winklig angeordnete lappenförmige Greifhilfe 6 angeordnet, die eine anteilig strukturierte Oberfläche 7 aufweist. Der eine Teil bzw. Schenkel der winkligen Greifhilfe 6 ist beabstandet und im Wesentlichen senkrecht zu dem den Abschluss der Stulpe 5 bildenden Rollrand auf dem Stulpenumfang angeordnet und erstreckt sich etwa über 50-70 % der Länge zwischen Rollrand und dem gegenüberliegenden Ende der Stulpe 5, das an den Handflächenraum 4 anschließt. Der zweite Teil bzw. Schenkel der winkligen Greifhilfe 6 ist in einem stumpfen Winkel von etwa 110° zu dem ersten Teil bzw. Schenkel angeordnet und erstreckt sich in Richtung des Daumens bzw. des Fingerraums 3, der an einen Daumen angepasst ist.

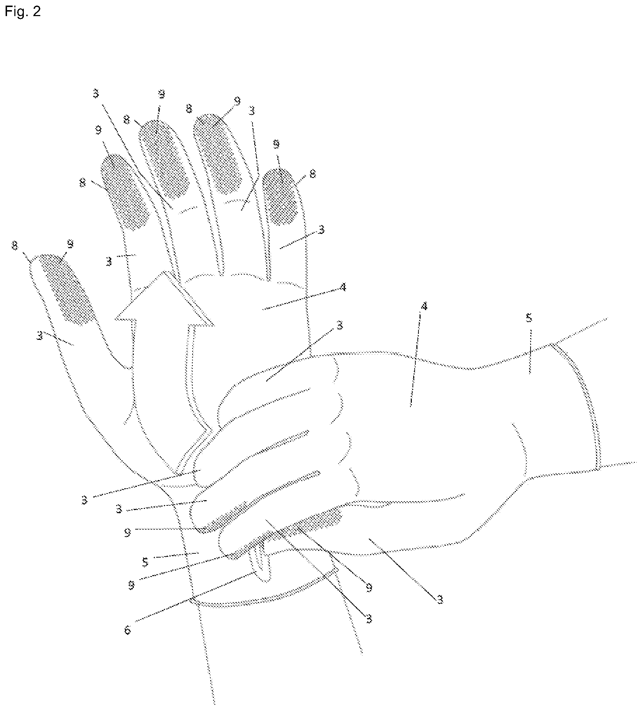

Figur 2 zeigt den Ausziehvorgang mit dem in Figur 1 gezeigten erfindungsgemäßen Handschuh 1. In der gezeigten Darstellung sind die Handschuhe für die rechte und die linke Hand nicht identisch und daher nicht beidseitig tragbar, da die Noppen, die die strukturierten Oberflächen 9 bilden, an den Enden 8 der Fingerräume 3 jeweils nur an einer Seite der Fingerräume 3 angeordnet sind und sich daher nur über ca. 20 bis 50 % des Umfangs der Enden eines jeden Fingerraums 3 erstrecken.

In der gezeigten Darstellung wird die Greifhilfe 6 des linken Handschuhs an ihrer strukturierten Oberfläche 7 so von einer rechten Hand ergriffen, dass die strukturierten Oberflächen 9 an den Enden 8 der Fingerräume 3 mit der strukturierten Oberfläche 7 der Greifhilfe 6 interagieren, d.h. für einen erhöhten Halt greifen die Noppen der strukturierten Oberfläche 7 mit den Noppen der strukturierten Oberflächen 9 ineinander.

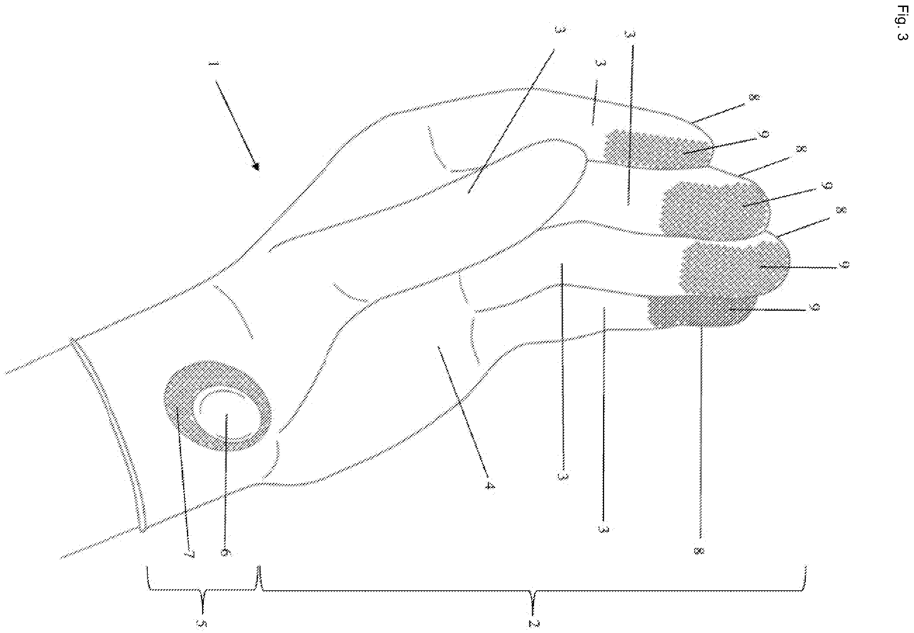

Figur 3 zeigt einen erfindungsgemäßen Handschuh mit einer knaufförmigen Greifhilfe 6, die eine strukturierte, in der gezeigten Darstellung genoppte, Oberfläche 7 aufweist. Die knaufförmige Greifhilfe 6 ist in der gezeigten Ausführungsform im Wesentlichen mittig unterhalb des Handflächenraums 4 auf dem Umfang der Stulpe 5 angeordnet. Entgegen der bevorzugten Ausführungsform, in der ein erfindungsgemäßer Handschuh beidseitig tragbar ist, ist der gezeigte Handschuh nur an der linken Hand tragbar, da die strukturierten Oberflächen 9 nur einseitig an den Enden 8 der Fingerräume 3 angeordnet sind, d.h. bei horizontal angeordnetem Handschuh mit nach links weisendem Daumen nur an der Oberseite der Fingerräume 3.

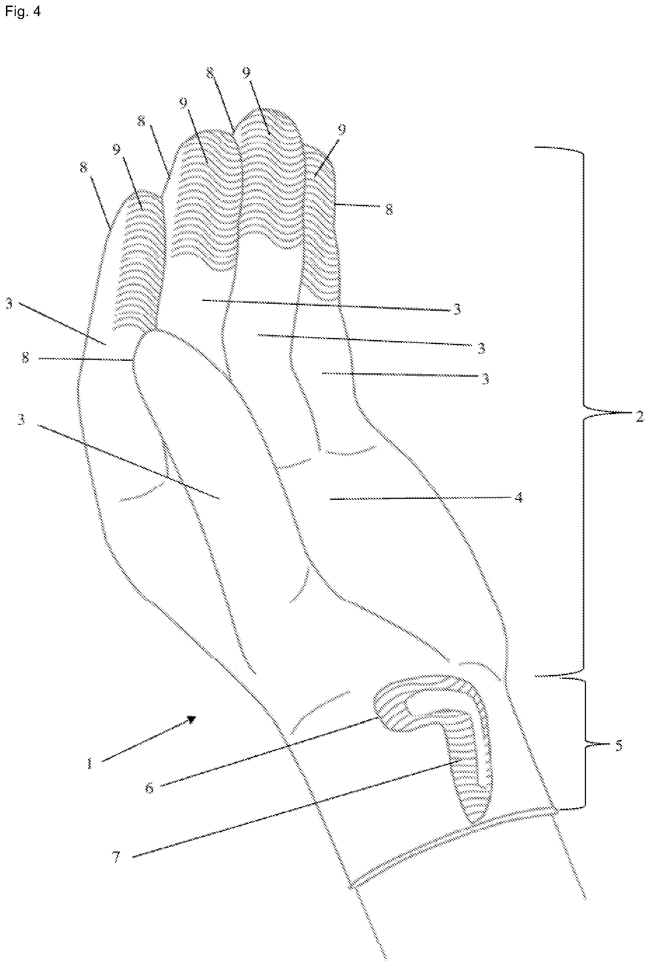

Die Figur 4 zeigt einen weiteren erfindungsgemäßen Handschuh 1 mit einem Handschuhkörper 2 und einer Stulpe 5. Der Handschuhkörper 2 weist fünf Fingerräume 3 auf, die jeweils mit dem Handflächenraum 4 verbunden sind. An ihren dem Handflächenraum 4 gegenüberliegenden Enden 8 weisen die Fingerräume 3 jeweils strukturierte Oberflächen 9 auf, die die Form von parallel zueinander beabstandeten Lamellen haben. Die strukturierten Oberflächen 9 sind teilumfänglich an den Enden 8 der Fingerräume 3 angeordnet und erstrecken sich über etwa die Hälfte bis ein Drittel der Länge der Fingerräume 3 in Richtung des Handflächenraums 4.
An der Stulpe 5 ist eine winklig angeordnete lappenförmige Greifhilfe 6 angeordnet, die ebenfalls im Wesentlichen parallel verlaufende Lamellen als strukturierte Oberfläche 7 aufweist.

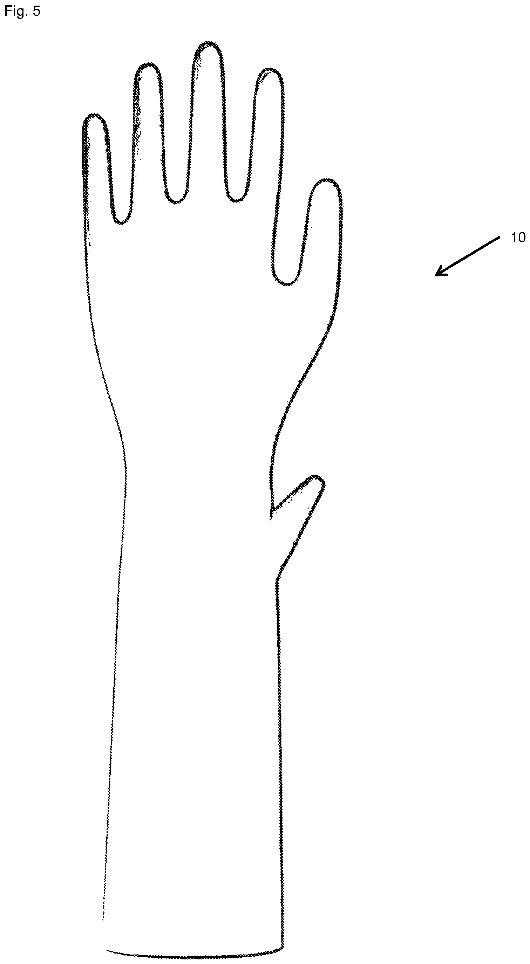

Die Figur 5 zeigt einen Formkörper 10. Ein solcher Formkörper ist in einem erfindungsgemäßen Verfahren zum Herstellen erfindungsgemäßer Handschuhe einsetzbar. Unterhalb des Daumens und seitlich des Handgelenks der Modellhand des Formkörpers weist dieser an seiner Oberfläche eine knaufähnliche bzw. kegelstumpfähnliche Erhebung auf. Beim Eintauchen des Formkörpers in ein Tauchbad wird daher die Greifhilfe an entsprechender Stelle, d.h. seitlich unterhalb des Fingerraums, der für die Aufnahme eines Daumens eingerichtet ist, direkt im formgebenden Prozess miterzeugt. Obwohl in der Figur 5 nicht gezeigt, soll der Formkörper an seiner Erhebung wie auch an zumindest zwei Fingern der Modellhand im Bereich der Fingerspitzen Strukturierungen aufweisen.

Die Figur 6 zeigt einen erfindungsgemäßen Handschuh mit einer Greifhilfe 6, die eine strukturierte Oberfläche 7 aufweist. Außerdem weist der Handschuh an jedem Fingerraum an deren dem Handflächenraum gegenüberliegenden Enden strukturierte Oberflächen 9 auf. Die Greifhilfe 6 ist auf ihrer dem Rollrand abgewandten Seite so auf dem Stulpenumfang angeordnet, dass der Übergang zwischen Stulpe 5 und Greifhilfe 6 ein U-förmiges Profil aufweist. Dieses U-förmige Profil weist entsprechend auch ein Formkörper 10 auf, der zur Herstellung des Handschuhs eingesetzt wurde. Aufgrund des U-förmigen Profils des Übergangs zwischen Stulpe 5 und Greifhilfe 6 entstehen beim Eintauchen des Formkörpers 10 in ein Tauchbad keine Luftblasen, wie sie beispielsweise bei einem eckigen Übergang zwischen Stulpe 5 und Greifhilfe 6 entstehen könnten. Auf ihrer dem Rollrand zugewandten Seite weist die Greifhilfe 6 ebenfalls eine leichte Mulde auf. Die entsprechende Mulde des Formkörpers 10, der zur Herstellung des Handschuhs eingesetzt wurde, bewirkt, dass sich beim Eintauchen des Formkörpers 10 in ein Tauchbad bzw. während des darauffolgenden Trocknens in dem erfindungsgemäßen Herstellungsverfahren vermehrt Flüssigkeit in der Mulde ansammelt. Der Handschuh weist daher bevorzugt in diesem Bereich eine erhöhte Materialdicke und daraus resultierend eine erhöhte Reißfestigkeit auf.

Generell ist es zudem bevorzugt, dass die Greifhilfe 6 einen z.B. winklig zu ihrer Längsrichtung angeordneten Vorsprung (nicht gezeigt) aufweist, an dem sich nach dem "dipping" bei vertikaler Haltung des Handschuhs mit nach unten weisenden Fingerspitzen bzw. Fingerräumen abfließende Flüssigkeit ansammelt, anstatt von der Greifhilfe 6 (z.B. auf den Fingerraum 3, der an einen Daumen angepasst ist) abzutropfen.

### Bezuqszeichenliste

- 1: Handschuh
- 2: Handschuhkörper
- 3: Fingerraum
- 4: Handflächenraum
- 5: Stulpe
- 6: Greifhilfe
- 7: strukturierte Oberfläche an Greifhilfe
- 8: dem Handflächenraum gegenüberliegendes Ende des Fingerraums
- 9: strukturierte Oberfläche an Handschuhkörper
- 10: Formkörper

## Patentansprüche

1. Handschuh (1) mit einem Handschuhkörper (2), der fünf Fingerräume (3) für die Finger einer Hand sowie einen mit jedem der Fingerräume (3) verbundenen Handflächenraum (4) aufweist, und einer an den Handflächenraum (4) anschließenden Stulpe (5), auf deren Umfang genau eine Greifhilfe (6) angeordnet ist, wobei der Handschuhkörper (2), die Stulpe (5) und die Greifhilfe (6) einteilig ausgebildet sind, die Greifhilfe (6) zumindest anteilig eine strukturierte Oberfläche (7) aufweist, und der Handschuhkörper (2) an zumindest zwei der Fingerräumen (3) an deren jeweiligen dem Handflächenraum (4) gegenüberliegenden Enden (8) eine strukturierte Oberfläche (9) aufweist, **dadurch gekennzeichnet, dass** die Greifhilfe (6) im angezogenen Zustand des Handschuhs seitlich des Handgelenks im Stulpenbereich angeordnet ist, wobei die Greifhilfe (6) eine Erhebung auf der Oberfläche der Stulpe (5) ist, die im angezogenen Zustand des Handschuhs (1) anders als der Rest des Handschuhs (1) nicht eng an der Hand anliegt, sondern von der Oberfläche des Handschuhs (1) absteht.

2. Handschuh (1) nach Anspruch 1, **dadurch gekennzeichnet, dass** der Handschuhkörper (2) an allen fünf Fingerräumen (3) an deren jeweiligen dem Handflächenraum (4) gegenüberliegenden Enden (8) eine strukturierte Oberfläche (9) aufweist.

3. Handschuh (1) nach einem der voranstehenden Ansprüche, **dadurch gekennzeichnet, dass** die strukturierte Oberfläche (7, 9) Noppen, Lamellen und/oder Rillen aufweist.

4. Handschuh (1) nach einem der voranstehenden Ansprüche, **dadurch gekennzeichnet, dass** die Stulpe (5) an ihrem dem Handschuhkörper (2) gegenüberliegenden Ende als Abschluss einen Rollrand aufweist, wobei die Wandstärke des Handschuhs in einem Bereich, der an die strukturierte Oberfläche (7) der Greifhilfe (6) angrenzt, im Vergleich zu einem angrenzenden Bereich ohne strukturierte Oberfläche und mit gleicher Entfernung zum Rollrand erhöht ist.

5. Handschuh (1) nach einem der voranstehenden Ansprüche, **dadurch gekennzeichnet, dass** sich die strukturierten Oberflächen (9) an den Enden (8) der Fingerräume (3) über 70 bis 100 % des Umfangs der Fingerräume (3) erstrecken.

6. Handschuh (1) nach einem der voranstehenden Ansprüche, **dadurch gekennzeichnet, dass** die strukturierte Oberfläche (9) an den Fingerräumen (3) und/oder die strukturierte Oberfläche (7) an der Greifhilfe (6) eine von dem restlichen Handschuh abweichende Farbe hat.

7. Verfahren zum Herstellen eines Handschuhs (1) nach einem der voranstehenden Ansprüche unter Verwendung eines Formkörpers (10), **dadurch gekennzeichnet, dass** die Greifhilfe (6) mit ihrer zumindest anteilig strukturierten Oberfläche (7) sowie die strukturierten Oberflächen (9) an den Fingerräumen (3) des Handschuhkörpers (2) im formgebenden Prozess des Handschuhs (1) miterzeugt werden.

8. Verfahren nach Anspruch 7, **gekennzeichnet durch** die Schritte
- Bereitstellung eines Formkörpers (10), der die Geometrie einer Hand mit einem daran anschließenden im Wesentlichen zylinderförmigen Abschnitt hat, auf dessen Umfang zumindest eine Erhebung angeordnet ist, wobei an der Oberfläche der Erhebung zumindest anteilig Strukturierungen angeordnet sind, und wobei an zumindest zwei Fingern der Modellhand jeweils an deren der Handfläche gegenüberliegenden Enden ebenfalls Strukturierungen angeordnet sind;
- Eintauchen des Formkörpers (10) in ein Tauchbad aus chemischen und/oder natürlichen Inhaltsstoffen;
- Trocknen des Handschuhs (1);
- Vulkanisieren des Handschuhs (1);
- Trennen des Handschuhs (1) von dem Formkörper (10).

9. Verwendung eines Handschuhs (1) nach einem der Ansprüche 1 bis 6 als Untersuchungs-, Operations- oder Arbeitshandschuh.

## Claims

1. Glove (1) having a glove body (2) which comprises five finger spaces (3) for the fingers of one hand as well as a palm space (4) connected to each of the finger spaces (3), and having a cuff (5) adjoining to the palm space (4), on the periphery of which cuff (5) exactly one gripping aid (6) is arranged, wherein the glove body (2), the cuff (5) and the gripping aid (6) are made in one piece, the gripping aid (6) at least partially has a structured surface (7) and the glove body (2) has a structured surface (9) at at least two of the finger spaces (3) at their respective ends (8) opposite the palm space (4), **characterized in that** the gripping aid (6) is arranged in the cuff region laterally of the wrist when the glove (1) is worn, wherein the gripping aid (6) is an elevation on the surface of the cuff (5), which, when the glove (1) is worn on the hand, unlike the rest of the glove (1) does not fit tight to the hand but is projecting from the surface of the glove (1).

2. Glove (1) according to claim 1, **characterized in that** the glove body (2) has a structured surface (9) at all five finger spaces (3) at their respective ends (8) opposite the palm space (4).

3. Glove (1) according to one of the preceding claims, **characterized in that** the structured surface (7, 9) comprises knobs, lamellae and/or grooves.

4. Glove (1) according to one of the preceding claims, **characterized in that** at its end opposite the glove body (2) the cuff (5) has a rolled edge as an end, wherein the wall thickness of the glove in a region adjacent to the structured surface (7) of the gripping aid (6) is increased in comparison to an adjacent region without a structured surface and with the same distance to the rolled edge.

5. Glove (1) according to one of the preceding claims, **characterized in that** the structured surfaces (9) at the ends (8) of the finger spaces (3) extend over 70 to 100 % of the periphery of the finger spaces (3).

6. Glove (1) according to one of the preceding claims, **characterized in that** the structured surface (9) at the finger spaces (3) and/or the structured surface (7) at the gripping aid (6) has a color which is different from the rest of the glove.

7. Process for producing a glove (1) according to one of the preceding claims using a former (10), **characterized in that** the gripping aid (6) with its at least partially structured surface (7) as well as the structured surfaces (9) at the finger spaces (3) of the glove body (2) are co-produced in the shaping process of the glove (1).

8. Process according to claim 7, **characterized by** the steps
- providing a former (10) having the geometry of a hand with an essentially cylindrical section adjoining thereto, at the periphery of which at least one elevation is arranged, wherein at the surface of the elevation patterning is arranged at least partially, and wherein at at least two fingers of the model hand patterning is arranged, too, at their respective ends opposite the palm;
- dipping the former (10) into an immersion bath of chemical and/or natural ingredients;
- drying the glove (1);
- vulcanizing the glove (1);
- separating the glove (1) from the former (10).

9. Use of a glove (1) according to one of the claims 1 to 6 as examination glove, surgical glove or work glove.

## Revendications

1. Gant (1) comportant un corps de gant (2), qui comporte cinq espaces de doigt (3) pour les doigts d'une main, ainsi qu'un espace de surface de main (4) raccordé à chacun des espaces de doigt (3), et comportant une manchette (5), se raccordant à l'espace de surface de main (4), et sur le pourtour de laquelle est disposée une aide à la préhension (6), le corps de gant (2), la manchette (5) et l'aide à la préhension (6) étant constitués d'une seule pièce, l'aide à la préhension (6) comportant au moins en partie une surface (7) structurée, et le corps de gant (2) comportant une surface (9) structurée sur au moins deux des espaces de doigt (3) à leurs extrémités (8) respectives opposées à l'espace de surface de main (4), **caractérisé en ce que**, lorsque le gant est enfilé, l'aide à la préhension (6) est disposée sur le côté du poignet dans la zone de manchette, l'aide à la préhension (6) étant une élévation sur la surface de la manchette (5) qui, lorsque le gant (1) est enfilé, n'est pas, à la différence du reste du gant (1), ajustée étroitement sur la main, mais est décollée de la surface du gant (1).

2. Gant (1) selon la revendication 1, **caractérisé en ce que**, sur tous les cinq espaces de doigt (3), à leurs extrémités (8) respectives opposées à l'espace de surface de main (4), le corps de gant (2) comporte une surface (9) structurée.

3. Gant (1) selon l'une des revendications précédentes, **caractérisé en ce que** la surface (7, 9) structurée comporte des protubérances, des lamelles et/ou des sillons.

4. Gant (1) selon l'une des revendications précédentes, **caractérisé en ce que**, à son extrémité opposée au corps de gant (2), la manchette (5) comporte une bande à rouler en tant que terminaison, l'épaisseur de paroi du gant étant, dans une zone qui est adjacente à la surface (7) structurée de l'aide à la préhension (6), surélevée par rapport à une zone adjacente sans surface structurée et à même distance de la bande à rouler.

5. Gant (1) selon l'une des revendications précédentes, **caractérisé en ce que**, aux extrémités (8) des espaces de doigt (3), les surfaces (9) structurées s'étendent sur 70 à 100 % du pourtour des espaces de doigt (3).

6. Gant (1) selon l'une des revendications précédentes, **caractérisé en ce que** la surface (9) structurée au niveau des espaces de doigt (3) et/ou la surface (7) structurée au niveau de l'aide à la préhension (6) a une couleur qui diffère du reste du gant.

7. Procédé de fabrication d'un gant (1) selon l'une des revendications précédentes avec utilisation d'un corps de formage (10), **caractérisé en ce que** l'aide à la préhension (6) avec sa surface (7) au moins partiellement structurée ainsi que les surfaces (9) structurées au niveau des espaces de doigt (3) du corps de gant (2) sont produites conjointement dans le processus de formage du gant (1).

8. Procédé selon la revendication 7, **caractérisé par** les étapes suivantes :
- fourniture d'un corps de formage (10) qui a la géométrie d'une main avec un tronçon essentiellement cylindrique qui s'y raccorde, sur le pourtour duquel il est disposé au moins une surélévation, des structurations au moins partielles étant disposées sur la surface de la surélévation, et des structurations étant également disposées sur au moins deux doigts de la main modèle, respectivement à leurs extrémités opposées à la surface de la main ;
- immersion du corps de formage (10) dans un bain de trempage constitué de composants chimiques et/ou naturels ;
- séchage du gant (1),
- vulcanisation du gant (1) ;
- séparation du gant (1) par rapport au corps de formage (10).

9. Utilisation d'un gant (1) selon l'une des revendications 1 à 6 en tant que gant d'examen, d'opération ou de travail.
